Europäisches Patentamt

European Patent Office

Office européen des brevets

(19)

(11) Publication number : **0 597 578 A2**

(12) # EUROPEAN PATENT APPLICATION

(21) Application number : 93307089.8

(22) Date of filing : 08.09.93

(51) Int. Cl.⁵ : **G01N 33/22**

(30) Priority : **11.09.92 GB 9219257**

(43) Date of publication of application :
**18.05.94 Bulletin 94/20**

(84) Designated Contracting States :
**DE FR IT NL**

(71) Applicant : **GEC-MARCONI LIMITED**
**The Grove, Warren Lane**
**Stanmore, Middlesex HA7 4LY (GB)**

(72) Inventor : **Perera, Guruge Elmo Lakshman**
**37 Meadow Way**
**Wembley, Middlesex HA9 7LB (GB)**

(74) Representative : **Cockayne, Gillian et al**
**The General Electric Company plc GEC Patent**
**Department Waterhouse Lane**
**Chelmsford, Essex CM1 2QX (GB)**

(54) **Energy measurement.**

(57)    Apparatus for measuring the calorific value of gaseous material, for example gaseous fuel, comprises a fuel cell $FC_1$, $FC_2$....$FC_n$ arranged to receive a sample of the gaseous material, and means 23 responsive to the open-circuit voltage of the fuel cell to determine the calorific value of the gaseous material from that voltage. A number of the fuel cells may be provided, each responsive to a different component of the gasous material.

Fig.1.

This invention relates to apparatus for measuring the chemical energy (calorific value) of gaseous material, such as gaseous fuel.

In Britain, calculation of the cost of gaseous fuel supplied to a consumer must take into account the calorific value of the fuel. The measurement of the calorific value should preferably be carried out periodically at each consumer's premises, and this requires the provision of cheap, small and reliable apparatus for measuring calorific value.

It is an object of the present invention to provide an improved apparatus for measuring the calorific value of gaseous material.

According to the invention there is provided apparatus for measuring the calorific value of gaseous material, comprising at least one fuel cell arranged to receive a sample of the gaseous material; and means responsive to the open-circuit voltage of the or each fuel cell to determine the calorific value of the gaseous material.

An embodiment of the invention will now be described, by way of example, with reference to the accompanying drawings, in which

Figure 1 is a schematic cross-sectional view of a fuel cell,

Figure 2 is a schematic diagram of a calorific value measuring apparatus in accordance with the invention, and

Figure 3 is a schematic pictorial view of a practical configuration of a fuel cell for use in the invention.

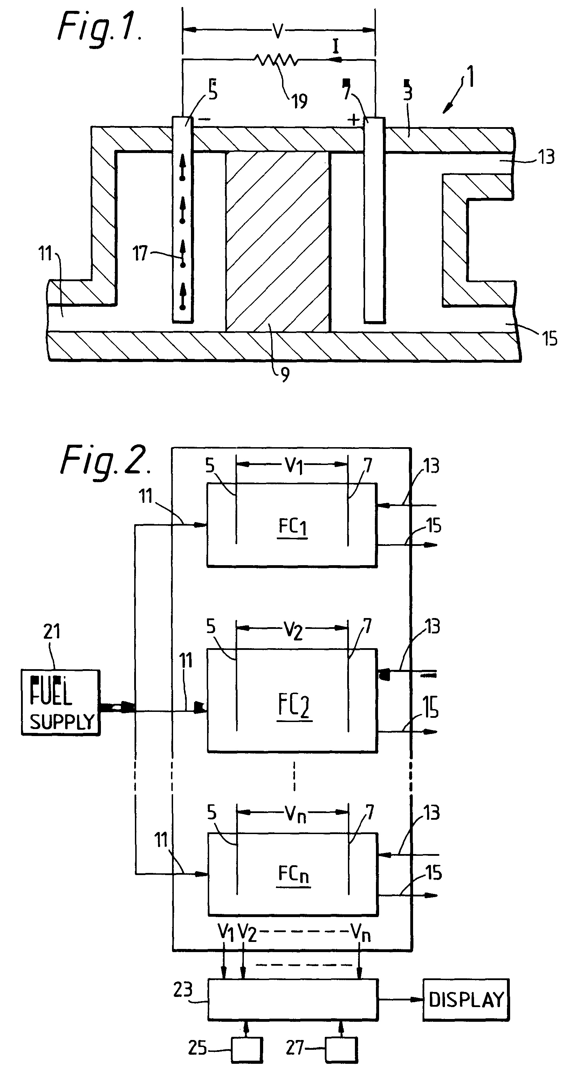

Referring to Figure 1, a conventional fuel cell 1 comprises a casing 3 containing a porous anode plate 5 and a porous cathode plate 7. An electrolyte 9 is contained between the electrode plates. A fuel inlet 11 is provided at the anode end of the casing. At the cathode end of the casing there are provided an inlet 13 for feeding oxidizing agent into the cell, and an outlet 15 for venting the products of the fuel cell reaction. In the combustion of the fuel gas fed into the inlet 11, the reaction involves the transfer of electrons from the fuel molecules to the molecules of the oxydizing agent which is fed into the inlet 13. The fuel gas diffuses through the anode and is oxidized, which releases electrons to the load circuit, as indicated by arrows 17.

In the conventional use of a fuel cell for producing an electric current, a load 19 is connected between the anode and cathode rods, so that a current I flows through the load.

In the present invention the load 19 is dispensed with, and the open-circuit (no-load) voltage of the fuel cell is measured. Current flow through the cell is virtually zero.

A single fuel cell may be used in the present invention, but a preferable arrangement comprising several fuel cells is shown in Figure 2. The calorific value measuring apparatus comprises fuel cells $FC_1$, $FC_2$---$FC_n$ to the inlet 9 of each of which a sample of gaseous fuel is fed from a supply 21. Air is fed into the inlet 13 of each cell, so that atmospheric oxygen acts as the oxidizing agent. The reaction products, comprising water and carbon dioxide, are vented to atmosphere through the outlets 15.

The fuel cells contain respectively different electrolytes and/or anode and cathode rod materials, so that the fuel cells handle different components of the gaseous fuel. Each fuel cell produces a respective open-circuit output voltage $V_1$, $V_2$---$V_n$ as a result of the reaction in the cell. The output voltages are fed to a microprocessor 23, which also receives signals from a gaseous fuel temperature sensor 25 and an inlet gas pressure sensor 27. The microprocessor determines the calorific value of the gaseous fuel from those input parameters.

The calorific value (heat of reaction) is determined in the following manner.

The calorific value H is given by

$$\Delta H = \frac{\Delta G}{\eta} = -2.305 \times 10^4 n \left( V_o - t \frac{dVo}{dt} \right)$$

$$\text{or } \Delta H = \frac{\Delta G}{\eta} = \frac{-2.305 \times 10^4 nV_o}{\eta} \text{ (calories)}$$

$$\text{or } \Delta H = \frac{-96943 nVo}{\eta} \text{ (Joules)} \quad (1)$$

where

$\Delta G$ = Gibbs free energy change for the cell reaction

n = number of moles of electrons involved at anode and cathode reaction of the fuel cell

$\eta$ = cell efficiency. This depends on the type of fuel and its composition and will lie between 0.9 and 1.0. For cell reactions for most types of fuels such as C0, $H_2$,$CH_4$, etc. occurring at 298K, $\eta$ can be ascertained by calibration techniques

and $V_o$ = open-circuit voltage of the fuel cell.

The constants $2.305 \times 10^4$ and 96943 are the Faraday Constant F expressed in different units, i.e.

$$F = 2.305 \times 10^4 \; \underline{\text{(cal.Coulombs)}}$$
$$\text{Joules}$$

$$= 96943 \text{ Coulombs}$$

F is the electrical charge of 1gm. mole of electrons (=6.02 x 10²³ electrons).

The definition of n appearing in equation (1) is clarified by considering the cell reaction as detailed below:

Anode reaction

$$v_1 C_1 + v_2 C_2 + \text{----} V_r C_r \rightarrow a_1 I_1^{2n1 \, (+)} + a_2 I_2^{2n2(+)} + a_s I_s^{2n2(+)} + 2(n_1, a_1 + n_2 a_2 + \text{----} n_s a_s)e^{-} \quad (2)$$

where v and a are stoichiometric coefficients

$C_1$, $C_2$---Cr chemical formulae for various components of the gaseous fuel

I = ions from the anode reaction

n = positive charge

Cathode reaction

$$\frac{n}{4} O_2 + ne^{-} \rightarrow \frac{n}{4} O_2^{2(-)} \quad (3)$$

Combining (2) and (3), the overall cell reaction is:

$$v_1 C_1 + v_2 C_2 + \ldots + v_r C_r + \frac{n}{4} O_2 \rightarrow a_1(I_1 O_{n1}) + a_1(I_1 O_{n1}) + a_2(I_2 O_{n2}) + \ldots + a_s(I_s O_{ns}) \quad (4a)$$

where,

$$n = 2(n_1 a_1 + n_2 a_2 + \ldots + n_s a_s)$$

By substituting

$$P_1 \text{ for } I_1 O_{n1}$$
$$P_2 \text{ for } I_2 O_{n2}$$
$$|$$
$$|$$
$$|$$
$$\text{and } P_s \text{ for } I_s O_{ns}$$

the reaction equation (4a) may be re-expressed as:

$$v_1 C_1 + v_2 C_2 + \ldots + v_r C_r + \frac{n}{4} O_2 \rightarrow a_1 P_1 + a_2 P_2 + \ldots + a_s P_s \quad (4b)$$

In the above $C_1, C_2, \ldots C_r$ represent the chemical formulae for the various chemical constituents of the gaseous fuel, which are oxidized to the products $P_1, P_2, \ldots P_s$. The latter symbols represent the chemical formulae of the products. $I_1, I_2 \ldots I_s$ represent positive ions formed at the anode, according to reaction equation (2), releasing n gm. moles of electrons. $e^{(-)}$ represents electron. In equation (1), H represents the heat of reaction for $v_1, v_2, \ldots v_r$ moles of the fuels $C_1, C_2, \ldots C_r$ present in the gaseous mixture. The change of Gibbs free energy $\Delta G$ for the cell reaction may be theoretically related to the fuel composition as indicated below. For the gaseous fuel mixture:

$$\Delta G = \Delta G^\circ + RT\log (\beta) \quad (5)$$

where

R = Universal Gas Constant (1989 Cal/K or 8314 Joules/K).

T = Absolute temperature of the fuel-cell operation, K

$$\beta = \frac{(P_1')^{a1}(P_2')^{a2} - - - (P_s')^{as}}{(P_1)^{v1}(P_2)^{v2} - - - (P_r)^{vr}} \quad (6)$$

$\Delta G^\circ$ = standard Gibbs free energy change for the cell reaction

$P_i'(i=1,2,\ldots s)$ = Partial pressures of fuel cell reaction The magnitude of $\Delta G^\circ$ may be evaluated from standard formations. Free energies of the fuel reactants and the products of cell reactions are as follows:

$$\Delta G^\circ = \sum_{i=1}^{s} a_1 \Delta' G^\circ_{fo} \sum_{i=1}^{r} v_i \Delta G^\circ_{fi}$$

3

$\Delta'G°_{fi}(i=1,2,....s)$ = Standard Formations Gibbs Free Energy of products $P_1, P_2,...P_s$ expressed as Cal/gm.mol of products or Joules/gm mole of products.

$\Delta G°_{fi}$, (i=1,2,...r) = Standard Formations Gibbs Free Energy of fuel reactants $C_1, C_2,...C_r$, expressed as Cal/gm or Joules/gm mole of fuel reactants.

Values of $\Delta'F°_{fi}$ and $\Delta G°_{fi}$ can be readily obtained by those skilled in the electrochemistry field. Hence, from equations (5),(6) and (7) it is possible to determine calorific values from $V_o$ values for any fuel cell.

As mentioned previously, a single fuel cell may be used, and it may be assumed that all of the fuel reactants undergo anode reaction and result in the production of all of the products $P_1$, $P_2$---$P_s$ at the cathode. In the preferred Figure 2 embodiment, which uses several fuel cells each handling a different fuel component $C_1$, $C_2$---$C_s$ and forming products $P_1, P_2$---$P_s$, the cell voltages $V_1, V_2$---$V_s$ are used by the microprocessor to the calorific value of the whole gas mixture.

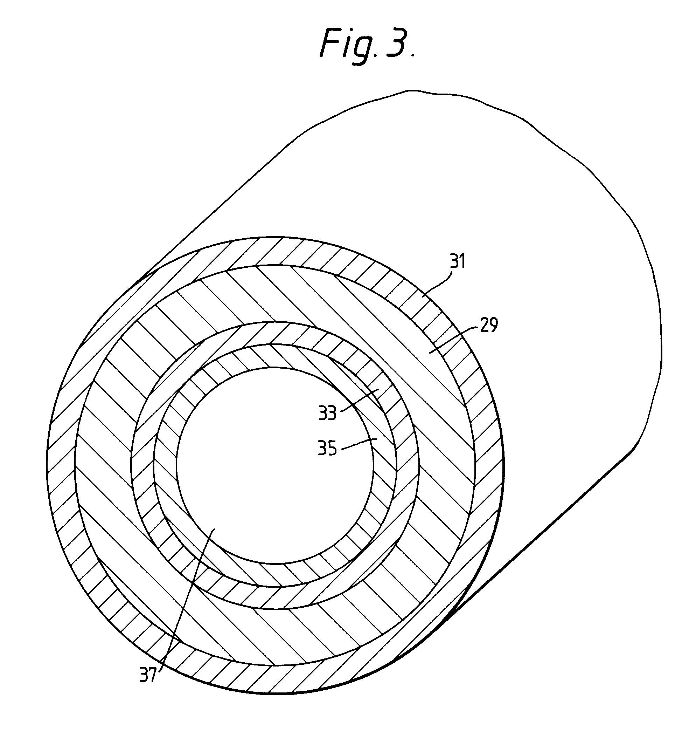

Each fuel cell may take the form shown schematically in Figure 3 of the drawings. The cell comprises a porous support tube 29 on the outer surface of which is provided a cathode layer 31. On the inner surface of the support tube is an electrolyte layer 33 with an anode layer 35 therein. Electrical output connections (not shown) are made to the electrode layers 31 and 35. In use of the fuel cell, the gaseous fuel under test flows through the bore 37 in contact with the anode layer 35, and the resulting open-circuit voltage is picked-off from the electrode layers. Ambient air in contact with the cathode layer 31 acts as the oxydizing agent.

The fuel cell is preferably of micron dimensions.

Problems of polarisation, which are usually important if a fuel cell is used for power production purposes, are not significant in the present invention, because the fuel cell is used only for open-circuit voltage measurement.

## Claims

1. Apparatus for measuring the calorific value of gaseous material, characterised by at least one fuel cell arranged to receive a sample of the gaseous material; and means responsive to the open-circuit voltage of the or each fuel cell to determine the calorific value of the gaseous material.

2. Apparatus as claimed in Claim 1, comprising a plurality of said fuel cells, each responsive to a different component of the gaseous material.

3. Apparatus as claimed in Claim 1, wherein the means to determine the calorific value comprises microprocessor means responsive to the open-circuit voltages of all of said fuel cells.

4. Apparatus as claimed in Claim 3, wherein the microprocessor is also responsive to the temperature and pressure of the gaseous material sample.

5. Apparatus as claimed in any preceding claim, wherein the or each fuel cell comprises a tube for conducting the gaseous material, the tube comprising concentric anode, electrolyte and cathode layers.

*Fig.1.*

*Fig.2.*

Fig. 3.